# EUROPÄISCHE PATENTSCHRIFT

(11) **EP 2 055 348 B1**
(45) Veröffentlichungstag und Bekanntmachung des Hinweises auf die Patenterteilung: **15.01.2014**
(21) Anmeldenummer: 08165504.5
(22) Anmeldetag: 30.09.2008
(51) Int. Cl.: A61N 1/372, G06F 19/00

(54) **Vorrichtung zur Bestimmung eines Nachsorgetermins für die Versorgung eines implantierbaren medizinischen Gerätes**
Device for calculating a maintenance appointment for supplying an implantable medical device
Dispositif de détermination d'un suivi thérapeutique pour le suivi d'un appareil médical pouvant être implanté

(30) Priorität: 30.10.2007 DE 102007051756
(43) Veröffentlichungstag der Anmeldung: 06.05.2009
(73) Patentinhaber: Biotronik CRM Patent AG, 6341 Baar (CH)
(72) Erfinder: Osche, Thomas, 12437 Berlin (DE)
(74) Vertreter: Lindner-Vogt, Karin L.

(56) Entgegenhaltungen:
- WO-A-2007/027570
- US-A1- 2001 025 137
- US-A1- 2003 028 082
- US-A1- 2003 144 711

## Beschreibung

Die Erfindung betrifft eine Vorrichtung zur Bestimmung eines Nachsorgetermins für ein medizinisches implantierbares Gerät, insbesondere für einen Elektrostimulator wie beispielsweise einen Herzschrittmacher, einen Cardioverter/Defibrillator oder dergleichen.

Es ist bekannt, implantierbare medizinische Geräte, wie beispielsweise Herzschrittmacher so zu gestalten, dass vom Herzschrittmacher erfasste oder vom Herzschrittmacher erzeugte Daten zu einer zentralen Serviceeinheit übermittelt werden. Die dazu notwendige Datenübertragung erfolgt beispielsweise drahtlos über eine vergleichsweise relativ kurze Reichweite von Implantat zu einem externen Gerät, welches sich zum Zwecke der Datenübertragung in Reichweite des Implantats befindet. Das externe Gerät fungiert quasi als Relaisstation und ist dazu ausgebildet, die vom Implantat ausgesandten Daten zu der zentralen Serviceeinheit weiter zu übertragen. In der zentralen Serviceeinheit können die Daten dann ausgewertet werden und einem Arzt zentral zur Verfügung stehen. Dies erlaubt im beschränkten Umfang Ferndiagnosen, die zum Teil persönliche Visiten des Arztes beim Patienten oder umgekehrt ersetzen können. Außerdem erlaubt es ein derartiges System, dass der Arzt frühzeitig möglicherweise kritische Gesundheitszustände erkennen kann, auf die er dann schnell reagieren kann, möglicherweise sogar bevor der Patient selbst einen kritischen Gesundheitszustand bemerkt.

Außerdem sind aus dem Stand der Technik verschiedene Systeme bekannt, die den Arzt bei der Nachsorge eines Patienten unterstützen. Beispiele sind in US 6,648,823, US 2003/028082 und US 2005/0065555 zu finden.

Auch ist aus der US Schrift US 2003/0144711 A1 ein System bekannt, bei dem ein implantierbares System, dass mit einem externen Gerät kommuniziert und überprüft, ob eine Nachsorgeuntersuchung geplant werden soll.

Die WO02/056235, die alle Merkmale des Oberbegriffs des Anspruchs 1 offenbart, offenbart ein System zum Vereinbaren eines Wartungstermins für ein medizinisches Gerät, speziell für eine Medikamentenpumpe.

Die US2004/0230115 A1 beschreibt ein Implantat zur Tumorüberwachung, insbesondere zur Ermittlung eines günstigen Zeitfensters für eine Behandlung.

Aufgabe der vorliegenden Erfindung ist es, eine verbesserte Vorrichtung zu finden, die eine Nachsorge eines Patienten unterstützt. Insbesondere ist eine Vorrichtung gewünscht, die den Arzt beim Finden eines geeigneten Nachsorgetermins unterstützt.

Die Aufgabe wird erfindungsgemäß durch eine Serviceeinheit gemäß Anspruch 1 gelöst.

Der Erfindung liegt die Erkenntnis zugrunde, dass die seitens des persönlichen medizinischen Gerätes empfangenen Daten genau die Informationen enthalten, die für die Bestimmung eines Nachsorgetermins relevant sind. Diese Daten enthalten üblicherweise neben gerätespezifischen Daten, die einen Aufschluss über den technischen Zustand des jeweiligen persönlichen medizinischen Gerätes geben, auch physiologische Daten, die seitens des persönlichen medizinischen Gerätes erfasst wurden und die einen Rückschluss auf den Gesundheitszustand eines jeweiligen Patienten zulassen. Wenn beispielsweise die Daten zu einem jeweiligen persönlichen medizinischen Gerät anzeigen, dass das persönliche medizinische Gerät für eine längere Zeit betriebsbereit sein dürfte und gleichzeitig die physiologischen Daten einen guten Gesundheitszustand des Patienten anzeigen, kann ein Nachsorgetermin in relativ weiter Zukunft gewählt werden. Wenn umgekehrt beispielsweise die Daten zu dem persönlichen medizinischen Gerät nahe legen, dass das persönliche medizinische Gerät selbst einer näheren Zuwendung bedarf, beispielsweise im Falle des Auftretens von Betriebsfehlern oder bei drohender Erschöpfung der Batteriekapazität oder wenn die physiologischen Daten anzeigen, dass der Gesundheitszustand des Patienten nicht so gut ist wie gewünscht, könnte ein kurzfristigerer Nachsorgetermin angezeigt sein.

Erfindungsgemäß ist somit vorgesehen, dass ein jeweiliger Nachsorgetermin individuell und in Abhängigkeit aktueller, vom jeweiligen persönlichen medizinischen Gerät stammender Daten durch die Nachsorgetermin-Bestimmungseinheit automatisch bestimmt wird. Es werden also in dem Sinne keine regelmäßig wiederkehrenden Intervalle durch die Nachsorgetermin-Bestimmungseinheit festgelegt. Vielmehr bestimmt die Nachsorgetermin-Bestimmungseinheit durch Bestimmung eines jeweils nächsten Nachsorgetermins lediglich das Intervall vom jeweiligen Augenblick bis zu diesem nächsten Nachsorgetermin.

Gemäß einer bevorzugten Ausführungsvariante besitzt die Serviceeinheit einen Speicher, in dem wenigstens ein vorangegangener Nachsorgetermin zu speichern ist. Die Serviceeinheit kann auch einen Zugriff auf solch einen Speicher besitzen. Die Nachsorgetermin-Bestimmungseinheit ist dabei ausgebildet, für die Bestimmung eines nächsten Nachsorgetermins das gespeicherte Datum eines vorangegangenen Nachsorgetermins zusätzlich zu den seitens des persönlichen medizinischen Gerätes empfangenen Daten zu berücksichtigen.

Die von der Nachsorgetermin-Bestimmungseinheit für die Bestimmung eines jeweils nächsten Nachsorgetermins herangezogenen Daten umfassen somit gemäß einer bevorzugten Ausführungsvariante zum einen wenigstens das Datum desjenigen Nachsorgetermins, der zuletzt bereits stattgefunden hat, sowie seitens des persönlichen medizinischen Gerätes empfangene Daten, nämlich vom persönlichen medizinischen Gerät erfasste physiologische Daten sowie Betriebsdaten zu dem persönlichen medizinischen Gerät.

Gemäß einer weiter bevorzugten Ausführungsvariante besitzt die Serviceeinheit eine zweite Schnittstelle als Schnittstelle für Benutzereingaben - im Folgenden auch Benutzereingabeeinheit genannt. Die Nachsorgetermin-Bestimmungseinheit ist dabei ausgebildet, Benutzereingaben entgegen zu nehmen und für die Bestimmung eines nächsten Nachsorgetermins ebenfalls zu verarbeiten. Dies erlaubt es unter anderem, dass ein jeweiliger Arzt Einfluss auf die Bestimmung eines nächsten Nachsorgetermins nehmen kann und beispielsweise ganz einfach den nächsten Nachsorgetermin noch einmal mit seinem persönlichen Kalender abgleichen kann. Auch hat der Arzt so die Möglichkeit, einen nächsten Nachsorgetermin auch von weiteren Umständen abhängig zu machen, die der Nachsorgetermin-Bestimmungseinheit nicht zugängig sind. Beispielsweise kann der Arzt bei der Bestimmung eines nächsten Nachsorgetermins auch den Umstand berücksichtigen, dass ein jeweiliger Patient eine besonders intensive ärztliche Betreuung wünscht.

In diesem Zusammenhang ist es besonders bevorzugt, wenn die Serviceeinheit zusammen mit der Nachsorgetermin-Bestimmungseinheit dazu ausgebildet ist, einen jeweils durch die Nachsorgetermin-Bestimmungseinheit bestimmten Nachsorgetermin zur Anzeige zu bringen und in Reaktion darauf eine Benutzereingabe entgegen zu nehmen, mit der ein Benutzer einen zur Anzeige gebrachten Nachsorgetermin entweder annimmt oder ablehnt und durch einen anderen vom Benutzer festgelegten Nachsorgetermin ersetzt. Mit zur Anzeige bringen von Daten für den nächsten Nachsorgetermin ist gemeint, dass die Serviceeinheit Daten generiert, die eine Anzeige eines nächsten Nachsorgetermins auf einem geeigneten Anzeigegerät erlauben. Zur Anzeige bringen kann in diesem Sinne auch das Aussenden einer Nachricht beispielsweise einer SMS aber gegebenenfalls auch einer akustischen Nachricht an einen jeweiligen Benutzer einschließen.

In Bezug auf die bevorzugte Ausführungsvariante, die es dem Arzt erlaubt, einen automatisch durch die Nachsorgetermin-Bestimmungseinheit bestimmten Nachsorgetermin durch einen selbstgewählten Nachsorgetermin zu ersetzen, ist anzumerken, dass auch in diesem Falle die vorangehende automatische Bestimmung eines nächsten Nachsorgetermins für den Arzt von großem Nutzen ist. Denn der durch die Nachsorgetermin-Bestimmungseinheit automatisch bestimmte nächste Nachsorgetermin enthält für den Arzt bereits einen Hinweis darauf, wie eilig oder weniger eilig eine persönliche Visite bei einem jeweiligen Patienten (oder umgekehrt) ist. Sollte der Arzt beispielsweise feststellen, dass er selbst einen deutlich abweichenden Nachsorgetermin vorgeschlagen hätte, kann er selbst noch einmal die von der Nachsorgebestimmungseinheit herangezogenen Daten in Augenschein nehmen, um sich von der seitens der Nachsorgetermin-Bestimmungseinheit beispielsweise erkannten Dringlichkeit noch einmal persönlich zu überzeugen. Darüber hinaus kann die Nachsorgetermin-Bestimmungseinheit so ausgebildet sein, dass sie benutzerbestimmte Nachsorgetermine relativ zum jeweiligen automatisch bestimmten Nachsorgetermin auswertet und typische Abweichungen zwischen dem benutzerbestimmten Nachsorgetermin und dem automatisch bestimmten Nachsorgetermin ermittelt und diese typischen Abweichungen speichert. Beispielsweise kann eine typische Abweichung darin bestehen, dass ein von einem Arzt jeweils in Antwort auf einen automatisch bestimmten Nachsorgetermin gewählter benutzerbestimmter Nachsorgetermin regelmäßig vor dem automatisch bestimmten Nachsorgetermin liegt, weil beispielsweise der Patient eine besonders intensive ärztliche Betreuung wünscht. Eine derartige, typische Abweichung kann von der Nachsorgetermin-Bestimmungseinheit automatisch ermittelt und gespeichert werden. Vorzugsweise ist die Nachsorgetermin-Bestimmungseinheit dann dazu ausgebildet, auch derart ermittelte typische Abweichungen für die Bestimmung eines automatisch bestimmten Nachsorgetermins zu berücksichtigen. Die Nachsorgetermin-Bestimmungseinheit "lernt" auf diese Weise beispielsweise, im Zusammenhang mit bestimmten persönlichen medizinischen Geräten (und damit im Zusammenhang mit bestimmten Patienten) regelmäßig eher kurzfristige Nachsorgetermine zu bestimmen.

Steht ein jeweiliger nächster Nachsorgetermin fest - entweder automatisch bestimmt oder gegebenenfalls durch den Benutzer geändert - stellt die Serviceeinheit diesen endgültigen Nachsorgetermin einem Benutzer zur Verfügung, vorzugsweise wird der endgültige Nachsorgetermin ausgedruckt. Dafür besitzt die Serviceeinheit gemäß einer bevorzugten Ausführungsvariante eine entsprechende dritte Schnittstelle.

Es ist darauf hinzuweisen, dass die vorgeschlagene Serviceeinheit mit der entsprechenden Nachsorgetermin-Bestimmungseinheit grundsätzlich nur eine unidirektionale Datenkommunikation von dem persönlichen medizinischen Gerät zu der Serviceeinheit erfordert. Selbstverständlich kann die erfindungsgemäße Serviceeinheit aber auch in einem System eingesetzt werden, bei dem eine bidirektionale Datenverbindung zwischen der Serviceeinheit und dem persönlichen medizinischen Gerät möglich ist.

Die Erfindung soll nun anhand eines Ausführungsbeispiels mit Bezug auf die Figuren näher erläutert werden. Die Figuren zeigen in:
- Fig. 1:: eine Übersicht über ein medizinisches Therapiesystem mit einem persönlichen medizinischen Gerät in Form eines Herzschrittmachers und einer Serviceeinheit in Form eines zentralen Servers;

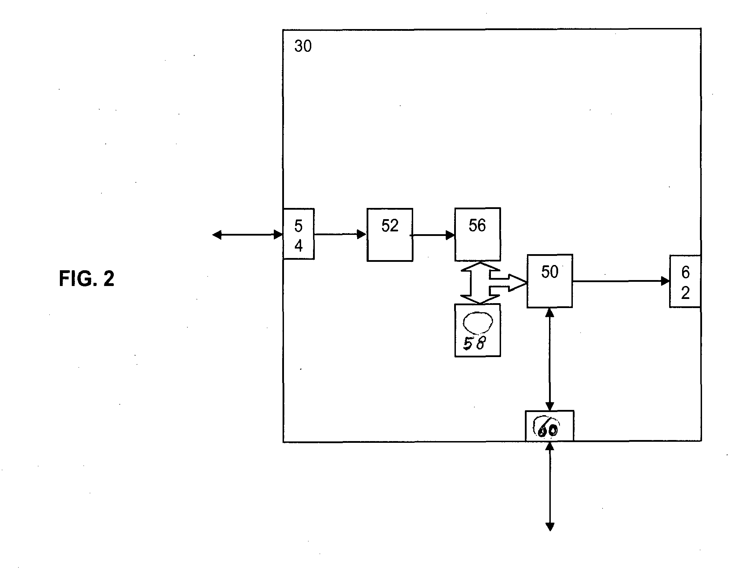

- Fig. 2:: ein schematisches Blockschaltbild einer erfindungsgemäßen Serviceeinheit.

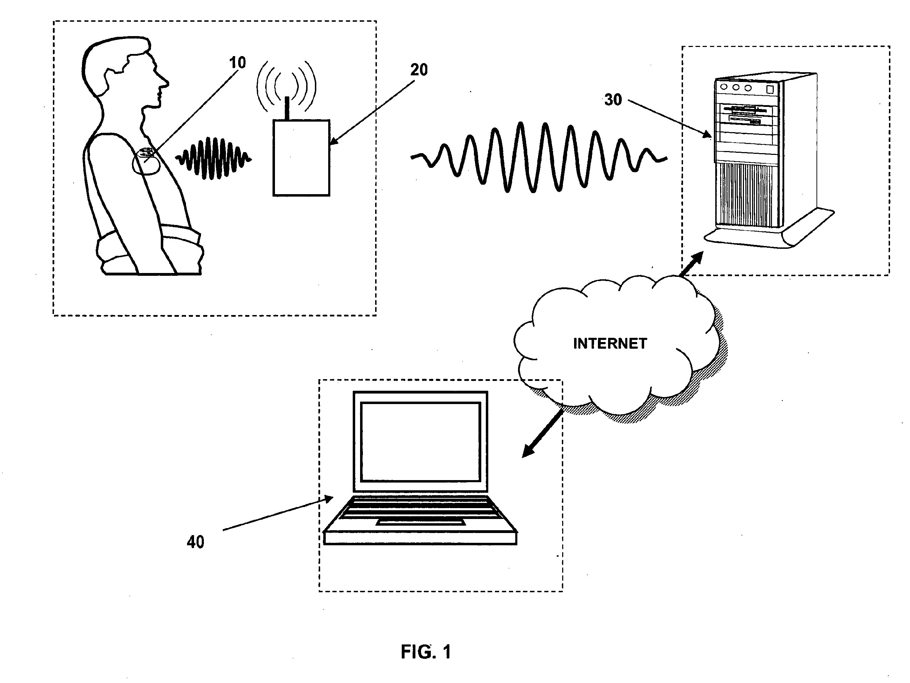

In Fig. 1 ist ein medizinisches Therapiesystem dargestellt, zu dem in erster Linie ein persönliches medizinisches Gerät 10 in Form eines implantierten Herzschrittmachers beziehungsweise implantierten Kardioverter/Defibrillator zählt. Das persönliche medizinische Gerät 10 kann wenigstens unidirektional mit einem externen Gerät 20 korrespondieren, um auf diese Weise Daten von dem persönlichen medizinischen Gerät zu dem externen Gerät 20 zu übertragen. In an sich bekannter Weise besitzt das persönliche medizinische Gerät 10 Sensoren zur Erfassung physiologischer Daten, beispielsweise zum Erfassen natürlicher Herzkontraktionen und daraus abgeleiteter Daten wie beispielsweise Daten bezüglich einer natürlichen Herzrate oder bezüglich des Vorliegens einer Fibrillation oder Ähnlichem. Außerdem ist das persönliche medizinische Gerät ausgebildet, auch Betriebsdaten über den eigenen Betriebszustand beispielsweise den Ladezustand seiner Batterien zu erfassen und diese Daten in regelmäßigen oder unregelmäßigen Abständen zu dem externen Gerät 20 zu übertragen.

Das externe Gerät 20 dient als Relaisstation und ist mit einer zentralen Serviceeinheit 30 verbunden. Die Datenverbindung zwischen dem externen Gerät 20 und der zentralen Serviceeinheit 30 kann dabei drahtgebunden oder drahtlos sein. Beispielsweise ist eine Anbindung via Virtual Private Network (VPN) möglich.

Die zentrale Serviceeinheit ist außerdem beispielsweise über das Internet mit einem Endgerät 40, beispielsweise einem persönlichen Computer eines Nutzers (Arztes) verbunden. Über das Endgerät 40 ist es möglich, seitens der zentralen Serviceeinheit 30 empfangene Daten anzuzeigen oder Benutzereingaben entgegenzunehmen und entsprechende Daten an die zentrale Serviceeinheit 30 zu übertragen. Die Besonderheit des dargestellten Systems besteht darin, dass die Serviceeinheit 30 dazu ausgebildet ist, automatisch einen Nachsorgetermin (Follow up Termin) zu bestimmen, zu dem sich Arzt und Patient für eine Nachsorgeuntersuchung begegnen sollten.

Zu diesem Zweck besitzt die Serviceeinheit 30 eine Nachsorgetermin-Bestimmungseinheit 50, die dazu ausgebildet ist, einen jeweiligen nächsten Nachsorgetermin auf Basis von Daten zu bestimmen, die die Serviceeinheit 30 seitens eines jeweiligen persönlichen medizinischen Gerätes 10 empfangen hat. Um derartige Daten zu empfangen, besitzt die Serviceeinheit 30 eine Datenschnittstelle 54 als erste Schnittstelle und eine mit dieser verbundenen Auswerteeinheit 52. Die Auswerteeinheit 52 ist dazu ausgebildet, die seitens des persönlichen medizinischen Gerätes 10 empfangenen Daten auszuwerten und entweder im ausgewerteten Zustand oder auch als Rohdaten in einem Speicher 56 zu speichern. Auf diesen Speicher 56 kann auch die Nachsorgetermin-Bestimmungseinheit 50 zugreifen.

Anhand der in dem Speicher 56 gespeicherten Daten bestimmt die Nachsorgetermin-Bestimmungseinheit 50 einen nächsten Nachsorgetermin. Die im Speicher 56 gespeicherten Daten sind vorzugsweise:
- Messdaten von elektrischen und nicht-elektrischen externen Sensoren des persönlichen medizinischen Geräts (Implantats) 10;
- Daten zur Systemintegrität von elektrischen und nicht-elektrischen externen Sensoren und elektrischen und nicht-elektrischen Aktoren des Implantats 10;
- Daten zur Systemintegrität des Implantats;
- Daten, die im Rahmen einer Modellbildung über den physiologischen Zustand eines Patienten im Implantat 10 oder außerhalb ermittelt wurden;
- Daten, die im Rahmen einer Modellbildung über den technischen Zustand des Implantats 10 im Implantat 10 oder außerhalb ermittelt wurden; und/oder
- Daten, die im Rahmen einer Modellbildung den technischen Zustand der elektrischen und nicht-elektrischen Aktoren und Sensoren des Implantats 10 im Implantat 10 ermittelt wurden.

Die im Rahmen einer Modellbildung ermittelten Daten werden vorzugsweise von der Auswerteeinheit 52 aus von Seiten des persönlichen medizinischen Gerätes empfangenen Rohdaten gewonnen. Alternativ kann eine Ermittlung von Daten im Rahmen einer Modellbildung auch bereits im persönlichen medizinischen Gerät 10 selbst erfolgen.

Vorzugsweise ist das persönliche medizinische Gerät (Implantat) 10 dazu ausgebildet, die entsprechenden Daten täglich an die zentrale Serviceeinheit 30 zu übertragen. Hierzu ist grundsätzlich nur eine unidirektionale Datenkommunikation zwischen dem persönlichen medizinischen Gerät 10 und dem zentralen Servicecenter 30 erforderlich. Im Falle einer möglichen, bidirektionalen Datenverbindung zwischen dem Implantat 10 und der zentralen Serviceeinheit 30 kann die Serviceeinheit 30 auch dazu ausgebildet sein, die entsprechenden Daten seitens des persönlichen medizinischen Gerätes 10 täglich abzufragen.

Die Nachsorgetermin-Bestimmungseinheit 50 ist darüber hinaus mit einem zweiten Speicher 58 verbunden, der dem Speichern vorangegangene Nachsorgetermine dient. Die beiden Speicher 56 und 58 können physikalisch ein und derselbe Speicher sein und somit verschiedene Speicherbereiche desselben physikalischen Speichers darstellen.

Anhand der in den Speichern 56 und 58 gespeicherten Daten ermittelt die Nachsorgetermin-Bestimmungseinheit 50 automatisch wenigstens einen nächsten Nachsorgetermin in dem Sinne, wie dies in der Beschreibungseinleitung erläutert ist.

Die den auf diese Weise jeweils automatisch bestimmten nächsten Nachsorgetermin repräsentierenden Daten werden über eine weitere Datenschnittstelle 60 so zur Verfügung gestellt, dass sie beispielsweise auf einer Anzeige durch einen Arzt zu betrachten sind. In dem Beispiel ist die Schnittstelle 60 vorzugsweise eine Schnittstelle zum Internet, die es erlaubt, dass ein jeweils automatisch bestimmter Nachsorgetermin auf der Anzeige beispielsweise des persönlichen Endgerätes 40 dargestellt wird.

Gleichzeitig ist die Nachsorge-Bestimmungseinheit so ausgebildet, dass sie eine Benutzereingabe in Reaktion auf das Anzeigen eines automatisch ermittelten Nachsorgetermins dergestalt zulässt, dass entweder der automatisch bestimmte Nachsorgetermin seitens des Benutzers (also des Arztes) bestätigt wird oder dass der Benutzer den automatisch bestimmten Nachsorgetermin ablehnt. Die Nachsorgetermin-Bestimmungseinheit 50 ist dazu ausgebildet, in Reaktion auf den letztgenannten Fall die manuelle Eingabe eines nächsten Nachsorgetermins über beispielsweise die Tastatur des persönlichen Endgerätes 40 zu erlauben.

Falls ein Benutzer den automatisch bestimmten Nachsorgetermin bestätigt, wird dieser automatisch bestimmte Nachsorgetermin als nächster Nachsorgetermin festgelegt, falls der Benutzer den automatisch bestimmten Nachsorgetermin ablehnt und durch einen von Hand einzugegebenen benutzerbestimmten Nachsorgetermin ersetzt, wird der benutzerbestimmte Nachsorgetermin als nächster Nachsorgetermin festgesetzt.

Die Nachsorgetermin-Bestimmungseinheit 50 speichert den auf diese Weise festgesetzten nächsten Nachsorgetermin in dem Speicher 58 und sorgt außerdem dafür, dass der festgesetzte nächste Nachsorgetermin durch einen Ausdruck protokolliert wird. Dazu ist die Nachsorgetermin-Bestimmungseinheit 50 mit einer entsprechenden Druckerschnittstelle 62 der Serviceeinheit 30 verbunden.

In diesem Zusammenhang ist die Nachsorgetermin-Bestimmungseinheit 50 weiterhin dazu ausgebildet, neben einem jeweiligen nächsten Nachsorgetermin gleichzeitig Kontaktdaten und gegebenenfalls weitere Daten zu einem jeweiligen Patienten auszudrucken, dem der Nachsorgetermin gilt. Dazu ist die Nachsorgetermin-Bestimmungseinheit 50 mit einer Patientendatendatenbank verbunden.

Eine Besonderheit der Nachsorgetermin-Bestimmungseinheit 50 besteht darin, dass sie ausgebildet ist, Benutzereingaben für die Bestimmung eines jeweiligen nächsten Nachsorgetermins zu berücksichtigen. Im einfachsten Fall sind dies die Benutzereingaben, mit denen ein Benutzer einen automatisch erstellten Nachsorgetermin ablehnt und statt dessen einen benutzerbestimmten Nachsorgetermin eingibt. Die Nachsorgetermin-Bestimmungseinheit 50 ist dazu ausgebildet, diese vom Benutzer eingegebenen Nachsorgetermine für sich genommen und in Bezug auf jeweils automatisch bestimmte Nachsorgetermine so auszuwerten, dass die Nachsorgetermin-Bestimmungseinheit 50 automatisch typische Muster erkennt. Ein derartiges Muster könnte beispielsweise darin bestehen, dass der von einem Arzt jeweils vorgegebene benutzerbestimmte Nachsorgetermin regelmäßig vor dem automatisch bestimmten Nachsorgetermin liegt. Die Nachsorgetermin-Bestimmungseinheit 50 ist für diesen Fall dazu ausgebildet, bei der automatischen Bestimmung eines nächsten Nachsorgetermins das so erkannte Muster zu berücksichtigen und einen früheren Nachsorgetermin zu bestimmen, als es ansonsten anhand der übrigen für die Bestimmung des jeweils nächsten Nachsorgetermins herangezogenen Daten indizieren.

Auf diese Weise ist die Nachsorgetermin-Bestimmungseinheit 50 dazu in der Lage, quasi die Vorlieben eines Arztes zu erlernen.

Letzteres wird besonders dann erleichtert, wenn die Nachsorgetermin-Bestimmungseinheit dazu ausgebildet ist, regelmäßig nicht nur einen einzigen nächsten Nachsorgetermin zu bestimmen, sondern beispielsweise eine Mehrzahl von Alternativen für einem nächsten Nachsorgetermin, unter denen der Arzt dann den jeweils ihm am passendeste erscheinende Nachsorgetermin auswählen kann. Falls sich aus dieser Auswahl regelmäßige Muster ergeben - beispielsweise regelmäßige Wahl des jeweils frühesten oder spätesten der angebotenen Nachsorgetermine -, können diese noch leichter bei der Bestimmung eines jeweils nächsten Nachsorgetermins oder bei der Bestimmung einer Mehrzahl jeweils nachfolgender Nachsorgetermine berücksichtigt werden.

## Patentansprüche

1. Serviceeinheit (30) mit
einer Schnittstelle (54) zum Empfang von Daten seitens eines persönlichen medizinischen Gerätes (10), wobei die Daten gerätespezifische Daten und physiologische Daten umfassen,
einer Auswerteeinheit (52) zum Auswerten seitens eines persönlichen medizinischen Gerätes (10) empfangener Daten, welche mit der Schnittstelle (54) verbunden ist,
sowie mit einer Nachsorgetermin-Bestimmungseinheit (50), die wenigstens indirekt mit der Auswerteeinheit (52) und/oder der Schnittstelle (54) verbunden ist und ausgebildet ist, wenigstens auf Basis seitens eines jeweiligen persönlichen medizinischen Gerätes (10) empfangener Daten einen jeweils nächsten Nachsorgetermin zu bestimmen und den bestimmten Nachsorgetermin auf einem geeigneten Anzeigegerät zur Anzeige zu bringen und in Reaktion darauf eine Benutzereingabe entgegen zu nehmen,
**dadurch gekennzeichnet, dass**
zur Anzeige bringen das Aussenden einer SMS an einen jeweiligen Benutzer umfasst,
die Nachsorgetermin-Bestimmungseinheit (50) ausgebildet ist, mehrere alternative nächste Nachsorgetermine automatisch zu bestimmen, und dass
die Serviceeinheit (30) ausgebildet ist, die alternativen Nachsorgetermine zur Anzeige zu bringen und über eine Benutzereingabeeinheit Benutzereingaben für die Auswahl eines der Nachsorgetermine oder die Ablehnung aller Nachsorgetermine entgegenzunehmen.

2. Serviceeinheit nach Anspruch 1, **dadurch gekennzeichnet, dass** die Serviceeinheit (30) einen Speicher (58) für wenigstens einen vergangenen Nachsorgetermin aufweist oder auf einen solchen Zugriff hat, wobei die Nachsorgetermin-Bestimmungseinheit ausgebildet ist, für die Bestimmung eines nächsten Nachsorgetermins das Datum eines vorangegangenen Nachsorgetermins zu berücksichtigen.

3. Serviceeinheit nach Anspruch 1 oder 2, **dadurch gekennzeichnet, dass** die Serviceeinheit (30) mit einer Benuterzeingabeeinheit verbunden oder zu verbinden ist und die Nachsorgetermin-Bestimmungseinheit (50) ausgebildet ist, Benutzereingaben entgegenzunehmen und für die Bestimmung eines nächsten Nachsorgetermins zu verarbeiten.

4. Serviceeinheit nach Anspruch 3, **dadurch gekennzeichnet, dass** die Serviceeinheit (30) ausgebildet ist, nach dem Bestimmen eines nächsten Nachsorgetermins durch die Nachsorgetermin-Bestimmungseinheit (50) diesen zur Anzeige zu bringen und eine Benutzereingabe entgegenzunehmen, mit der ein Benutzer einen zur Anzeige gebrachten Nachsorgetermin annimmt oder ablehnt.

5. Serviceeinheit nach Anspruch 3 oder 4, **dadurch gekennzeichnet, dass** die Serviceeinheit (30) ausgebildet ist, nach dem Ablehnen eines von der Nachsorgetermin-Bestimmungseinheit (50) bestimmten Nachsorgetermins eine Eingabe eines benutzerbestimmten Nachsorgetermins entgegenzunehmen und diesen zu speichern.

6. Serviceeinheit nach Anspruch 5, **dadurch gekennzeichnet, dass** die Nachsorgetermin-Bestimmungseinheit (50) ausgebildet ist, gespeicherte benutzerbestimmte Nachsorgetermine relativ zu jeweiligen von der Nachsorgetermin-Bestimmungseinheit (50) bestimmten Nachsorgeterminen derart auszuwerten, dass die Nachsorgetermin-Bestimmungseinheit typische Abweichungen zwischen den von ihr bestimmten Nachsorgeterminen und den benutzerbestimmten Nachsorgeterminen erfasst und für die zukünftige Bestimmung eines Nachsorgetermins berücksichtigt.

7. Medizinisches Therapiesystem mit einer Serviceeinheit gemäß einem der Ansprüche 1 bis 6 und einem persönlichen medizinischen Gerät in Form eines implantierbaren Elektrostimulationsgerätes.

8. Medizinisches Therapiesystem nach Anspruch 7, **dadurch gekennzeichnet, dass** das implantierbare Elektrostimulationsgerät ein Herzschrittmacher und/oder ein Kardioverter/Defibrillator ist.

## Claims

1. A service unit (30), comprising:
an interface (54) for receiving data from a personal medical device (10), the data including device-specific data and physiological data;
an evaluation unit (52) for evaluating data received from a personal medical device (10), the unit being connected to the interface (54);
and a follow-up appointment determination unit (50), which is at least indirectly connected to the evaluation unit (52) and/or to the interface (54) and which is designed to determine a respective next follow-up appointment, at least based on data received from a respective personal medical device (10), and to display the determined follow-up appointment on a suitable display device and, in response thereto, to receive a user input,
**characterized in that** the displaying includes the emission of a text message to a respective user, the follow-up determination unit (50) is designed to automatically determine multiple alternative next follow-up appointments, and the service unit (30) is designed to display the alternative follow-up appointments and to accept, via a user input unit, user inputs for the selection of one of the follow-up appointments or the rejection of all follow-up appointments.

2. The service unit according to claim 1, **characterized in that** the service unit (30) comprises a memory (58) for at least one past follow-up appointment, or has access to the same, wherein the follow-up appointment determination unit is designed to consider the date of a preceding follow-up appointment when determining a next follow-up appointment.

3. The service unit according to claim 1 or 2, **characterized in that** the service unit (30) is connected, or is to be connected, to a user input unit, and the follow-up appointment determination unit (50) is designed to receive user inputs and process these for the determination of a next follow-up appointment.

4. The service unit according to claim 3, **characterized in that** the service unit (30) is designed, after a next follow-up appointment has been determined by the follow-up appointment determination unit (50), to display this appointment and to receive a user input with which a user accepts or rejects a follow-up appointment that is displayed.

5. The service unit according to claim 3 or 4, **characterized in that** the service unit (30) is designed, after a follow-up appointment determined by the follow-up appointment determination unit (50) was rejected, to receive an input of a user-determined follow-up appointment and to store the same.

6. The service unit according to claim 5, **characterized in that** the follow-up appointment determination unit (50) is designed to evaluate stored user-determined follow-up appointments relative to the respective follow-up appointments determined by the follow-up appointment determination unit (50) in such a way that the follow-up appointment determination unit captures typical deviations between the follow-up appointments determined by it and the user-determined follow-up appointments and takes these into consideration for the future determination of a follow-up appointment.

7. A medical treatment system, comprising a service unit according to any one of claims 1 to 6 and a personal medical device in the form of an implantable electrostimulation device.

8. The medical treatment system according to claim 7, **characterized in that** the implantable electrostimulation device is a cardiac pacemaker and/or a cardioverter/defibrillator.

## Revendications

1. Unité de service (30) avec
une interface (54) pour la réception de données par un dispositif médical personnel (10), dans laquelle les données comprennent des données spécifiques au dispositif et des données physiologiques,
une unité d'évaluation (52) reliée à l'interface (54), pour l'évaluation des données reçues par un dispositif médical personnel (10),
et avec une unité de détermination de date de suivi (50) reliée au moins indirectement à l'unité d'évaluation (52) et/ou à l'interface (54) et conçue pour déterminer une prochaine date de suivi au moins sur la base des données reçues par un dispositif médical personnel (10) correspondant, et pour afficher la date de suivi déterminée sur un dispositif d'affichage approprié, et pour réceptionner une entrée d'utilisateur en réponse à cela, **caractérisée en ce que** l'affichage comprend l'émission d'un SMS à un utilisateur respectif, **en ce que** l'unité de détermination de date de suivi (50) est conçue pour déterminer automatiquement plusieurs prochaines dates de suivi alternatives, et **en ce que** l'unité de service (30) est conçue pour afficher les dates de suivi alternatives et pour réceptionner des entrées d'utilisateur pour la sélection d'une date de suivi ou le rejet de toutes les dates de suivi, par le biais d'une unité de saisie d'utilisateur.

2. Unité de service selon la revendication 1, **caractérisée en ce que** l'unité de service (30) comporte une mémoire (58) pour au moins une date de suivi passée, ou a accès à une telle mémoire, dans laquelle l'unité de détermination de date de suivi est conçue pour tenir compte d'une date de suivi précédente lors de la détermination d'une prochaine date de suivi.

3. Unité de service selon la revendication 1 ou 2, **caractérisée en ce que** l'unité de service (30) est reliée ou susceptible d'être reliée à une unité de saisie d'utilisateur, et l'unité de détermination de date de suivi (50) est conçue pour réceptionner les entrées d'utilisateur et pour les traiter en vue de la détermination d'une prochaine date de suivi.

4. Unité de service selon la revendication 3, **caractérisée en ce que** suite à la détermination d'une prochaine date de suivi par l'unité de détermination de date de suivi (50), l'unité de service (30) est conçue pour afficher celle-ci et pour réceptionner une entrée d'utilisateur avec laquelle un utilisateur accepte ou rejette une date de suivi affichée.

5. Unité de service selon la revendication 3 ou 4, **caractérisée en ce que** l'unité de service (30) est conçue pour réceptionner une entrée relative à une date de suivi déterminée par l'utilisateur et pour stocker celle-ci, suite au rejet d'une date de suivi déterminée par l'unité de détermination de date de suivi (50).

6. Unité de service selon la revendication 5, **caractérisée en ce que** l'unité de détermination de date de suivi (50) est conçue pour évaluer des dates de suivi déterminées par l'utilisateur et stockées, par rapport aux différentes dates de suivi déterminées par l'unité de détermination de date de suivi (50), de manière à ce que l'unité de détermination de date de suivi (50) puisse détecter des écarts typiques entre les dates de suivi déterminées par elle-même et les dates de suivi déterminées par l'utilisateur, et ensuite en tenir compte dans la détermination future d'une date de suivi.

7. Système thérapeutique médical avec une unité de service selon l'une des revendications 1 à 6, et avec un dispositif médical personnel sous la forme d'un dispositif de stimulation électronique implantable.

8. Système thérapeutique médical selon la revendication 7, **caractérisé en ce que** le dispositif de stimulation électronique implantable est un stimulateur cardiaque et/ou un cardioverteur/défibrillateur.
